# EUROPEAN PATENT SPECIFICATION

(11) **EP 2 238 160 B1**
(45) Date of publication and mention of the grant of the patent: **22.06.2016**
(21) Application number: 09705591.7
(22) Date of filing: 27.01.2009
(51) Int. Cl.: C07K 14/47, A61K 39/385, C12N 15/62

(54) **MULTIMERIC PROTEINS ABLE TO INDUCE AN ANTIBODY RESPONSE AGAINST THE BETA-AMYLOID AND USE THEREOF**
MULTIMERE PROTEINE ZUR INDUKTION EINER ANTIKÖRPERANTWORT GEGEN BETA-AMYLOID UND DEREN VERWENDUNG
PROTÉINES MULTIMÈRES CAPABLES D'INDUIRE UNE RÉPONSE D'ANTICORPS À L'ENCONTRE DU BÊTA-AMYLOÏDE ET LEUR UTILISATION

(30) Priority: 28.01.2008 IT NA20080006
(43) Date of publication of application: 13.10.2010
(73) Proprietor: CONSIGLIO NAZIONALE DELLE RICERCHE, 00185 Roma (IT)
(72) Inventor: PRISCO, Antonella, 00185 Roma (IT); DE BERARDINIS, Piergiuseppe, 00185 Roma (IT); DE FALCO, Diana, 80058 Torre Annunziata (NA) (IT); CAIVANO, Antonella, 85050 Tito (PZ) (IT)
(74) Representative: Comoglio, Elena
(86) International application number: PCT/IB2009/050329
(87) International publication number: WO 2009/095857

(56) References cited:
- WO-A-2007/027640
- US-A1- 2005 152 878
- DOMINGO G J ET AL: "MULTIPLE DISPLAY OF PEPTIDES AND PROTEINS ON A MACROMOLECULAR SCAFFOLD DERIVED FROM A MULTIENZYME COMPLEX" JOURNAL OF MOLECULAR BIOLOGY, LONDON, GB, vol. 305, no. 2, 12 January 2001 (2001-01-12), pages 259-267, XP000998078 ISSN: 0022-2836 cited in the application
- ESPOSITO ET AL: "Immunogenicity and therapeutic efficacy of phage-displayed beta-amyloid epitopes" MOLECULAR IMMUNOLOGY, PERGAMON, GB, vol. 45, no. 4, 31 October 2007 (2007-10-31), pages 1056-1062, XP022322732 ISSN: 0161-5890 cited in the application
- KIM ET AL: "Induction of anti-inflammatory immune response by an adenovirus vector encoding 11 tandem repeats of Abeta1-6: Toward safer and effective vaccines against Alzheimer's disease" BIOCHEMICAL AND BIOPHYSICAL RESEARCH COMMUNICATIONS, ACADEMIC PRESS INC. ORLANDO, FL, US, vol. 336, no. 1, 14 October 2005 (2005-10-14), pages 84-92, XP005015289 ISSN: 0006-291X
- CHACKERIAN B ET AL: "Virus and virus-like particle-based immunogens for Alzheimer's disease induce antibody responses against amyloid-beta without concomitant T cell responses" VACCINE, BUTTERWORTH SCIENTIFIC. GUILDFORD, GB, vol. 24, no. 37-39, 11 September 2006 (2006-09-11), pages 6321-6331, XP025151671 ISSN: 0264-410X [retrieved on 2006-09-11] cited in the application
- WEINER H L ET AL: 'Inflammation and therapeutic vaccination in CNS diseases' NATURE vol. 420, no. 6917, 19 December 2002, pages 879 - 884, XP055206334 DOI: 10.1038/nature01325 ISSN: 0028-0836

## Description

### TECHNICAL FIELD OF THE INVENTION

The present invention relates to molecules with multimeric structure i.e. multimeric proteins based on the E2 component of pyruvate dehydrogenase, compositions comprising them and uses thereof to stimulate an immune response against the beta amyloid peptide for the prevention and/or treatment of Alzheimer's disease.

### STATE OF THE ART

Alzheimer's disease, which affects over 12 million people throughout the world, is characterized by the deposition, in the brain, of insoluble protein aggregates, the amyloid plaques (Morgan et al., 2004). The main constituent of the plaques is the beta amyloid peptide (Aβ), which derives from the proteolytic processing of the APP (Amyloid precursor protein) membrane protein (Morgan et al., 2004). In animal models of Alzheimer's disease, it has been demonstrated that immunization with the beta amyloid peptide can reduce the quantity of cerebral amyloid and the progression of the pathology.

This effect is mediated by anti beta amyloid antibodies. (Morgan and Gitter, 2004; Weksler et al., 2005) For the development of immunotherapy protocols of Alzheimer's disease, two important problems must be solved: the poor immunogenicity of the beta amyloid peptide, and the potential dangerousness of cell-mediated self-immune responses against the beta amyloid. In a clinical immunization trial of patients affected by Alzheimer's diseases with the entire beta amyloid peptide pre-aggregated, out of 300 individuals who received the vaccine only 59 exhibited an antibody response against beta amyloid with a titer above 1 :2200, and 6% of patients developed an adverse inflammatory reaction, characterized by the infiltration of T lymphocytes in the brain (Gilman et al., 2005).

Anti-amyloid vaccines that consist of B epitopes of beta amyloid physically bound to a carrier that includes exogenous T epitopes are potentially more immunogenic and less dangerous for man than vaccines that contain T epitopes of beta amyloid (and hence autologous), that can trigger a cell-mediated self-immune reaction. The intensity of the humoral response to a recombinant vaccine based on the fusion of a carrier protein and of a specific epitope depends on the carrier, on the selected epitope, and also on the amino acidic residues that flank the epitope in the recombinant protein (Esposito et al., 2008), which makes it important to screen new constructs for immunogenicity optimization. The carrier used also influences the isotype of the antibody response (Chackerian at al., 2006), which is a correlate of the TH1/TH2 polarization of the T lymphocytes during the immune response. In the case of Alzheimer's disease, it has been hypothesized that vaccines that trigger a TH2 immune response are less dangerous than vaccines that trigger a TH1 (pro-inflammatory) response (Kim et al., 2007).

Esposito et al., 2008 discloses the immunogenicity of different regions of beta amyloid, including the Aβ (2-6) epitope, displayed on a filamentous phage.

### DESCRIPTION OF THE INVENTION

The present invention as described in the claims consists of fusion proteins that contain an epitope B of beta amyloid (in particular, the epitope 1-11, DAEFRHDSGYE (SEQ ID NO. 6) or the epitope 2-6, AEFRH (SEQ ID NO.5) and the catalytic domain of the E2 component of pyruvate dehydrogenase of *Geobacillus stearothermophilus* with the sequence:

PGPAAAEEKAAPAAAKPATTEGEFPETREKMSGIRRAIAKAMVHSKHTAPHVTLM
DEADVTKLVAHRKKFKAIAAEKGIKLTFLPYVVKALVSALREYPVLNTSIDDETEE
IIQKHYYNIGIAADTDRGLLVPVIKHADRKPIFALAQEINELAEKARDGKLTPGEMK
GASCTITNIGSAGGQWFTPVINHPEVAILGIGRIAEKPIVRDGEIVAAPMLALSLSFD
HRMIDGATAQKALNHIKRLLSDPELLLMEA (SEQ ID NO. 7).

These recombinant proteins, expressed in *Escherichia coli* and purified, self-assemble *in vitro* to form the virus-like particles Aβ(1-11)E2₆₀ₘₑᵣ and Aβ(2-6)E2₆₀ₘₑᵣ which are used to trigger an antibody response against beta amyloid in laboratory mice. The antibody response obtained after two injection of the virus-like particles reaches the titer of 1:96000 when the Aβ(1-11)E2₆₀ₘₑᵣ particles are used, and the titer 1:12000 when the Aβ(2-6)E2₆₀ₘₑᵣ particles are used. In the anti-beta amyloid antibodies obtained in these immunizations, a prevalence of the IgG1 isotype with respect to the IgG2a isotype is observed, an isotype ratio that is indicative of a TH2 immune
response. The proteins of the invention then enable to induce an antibody response against beta amyloid, with a high antibody titer, and a prevalence of the IgG1 isotype over the IgG2a isotype, indicative of a TH2 immune reaction. The proteins of the invention are useful for the development of vaccines against Alzheimer's disease and against amyloid deposition disease.

To generate the virus-like particles Aβ(1-11)E2₆₀ₘₑᵣ and Aβ(2-6)E2₆₀ₘₑᵣ the authors exploited an antigen presentation system based on the pyruvate dehydrogenase complex of Geobacillus stearothermophilus (Domingo G.J., Perham R.N., EP1244775; WO0142439). Two of the enzymes that comprise it, E1 and E3, assemble on the surface of a large protein frame formed by the E2 enzyme, a dihydrolipoyl acyltransferase. The C-terminal catalytic domain of each E2 monomer is able to self-assemble in trimers, which in turn aggregate to form a 60mer with icosahedral symmetry. The 60mer has molecular weight above 1.5MDa and a diameter of 24nm, viewable with the electronic microscope. Starting from denaturizing conditions, E2 can be renaturized in vitro to form the 60mer, with no need for chaperonins. An effective refolding of E2 can be obtained, with formation of 60mers, even when extraneous peptides or proteins are inserted at the N-terminal of the catalytic domain instead of the natural peripheral domains of E2. Then, the core domain of E2, (E2DISP: PGPAAAEEKAAPAAAKPATTEGEFPETREKMSGIRRAIAKAMVHSKHTAPHVTLMDE ADVTKLVAHRKKFKAIAAEKGIKLTFLPYVVKALVSALREYPVLNTSIDDETEEIIQKH YYNIGIAADTDRGLLVPVIKHADRKPIFALAQEINELAEKARDGKLTPGEMKGASCTIT NIGSAGGQWFTPVINHPEVAILGIGRIAEKPIVRDGEIVAAPMLALSLSFDHRMIDGAT AQKALNHIKRLLSDPELLLMEA (SEQ ID NO. 7), appropriately engineered, can expose up to 60 pairs of an exogenous polypeptide on the surface of a scaffold with high molecular weight (Domingo GJ, et al., 2001).

The authors engineered E2DISP to obtain the protein Aβ(2-6)E2 (MAEFRHPGPAAAEEKAAPAAAKPATTEGEFPETREKMSGIRRAIAKAMVHSKHTAP HVTLMDEADVTKLVAHRKKFKAIAAEKGIKLTFLPYVVKALVSALREYPVLNTSIDD ETEEIIQKHYYNIGIAADTDRGLLVPVIKHADRKPIFALAQEINELAEKARDGKLTPGE MKGASCTITNIGSAGGQWFTPVINHPEVAILGIGRIAEKPIVRDGEIVAAPMLALSLSFD HRMIDGATAQKALNHIKRLLSDPELLLMEA, SEQ ID NO.8), which expresses at the N-terminal, immediately after the initial methionine, the epitope 2-6 of the beta amyloid 1-42 (DAEFRHDSGYEVHHQKLVFFAEDVGSNKGAIIGLMVGGVVIA, SEQ ID NO. 9) i.e. the amino acidic sequence AEFRH (SEQ ID NO. 5), and to obtain the protein Aβ(1-11)E2 (MDAEFRHDSGYEPGPAAAEEKAAPAAAKPATTEGEFPETREKMSGIRRAIAKAMVH SKHTAPHVTLMDEADVTKLVAHRKKFKAIAAEKGIKLTFLPYVVKALVSALREYPVL NTSIDDETEEIIQKHYYNIGIAADTDRGLLVPVIKHADRKPIFALAQEINELAEKARDGK LTPGEMKGASCTITNIGSAGGQWFTPVINHPEVAILGIGRIAEKPIVRDGEIVAAPMLAL SLSFDHRMIDGATAQKALNHIKRLLSDPELLLMEA, SEQ ID NO. 10), which expresses at the N-terminal, immediately after the initial methionine, the epitope 1-11 of the beta amyloid, i.e. the amino acidic sequence DAEFRHDSGYE (SEQ ID NO. 6).

The proteins Aβ(2-6)E2 and Aβ(1-11)E2, in purified form, spontaneously formed *in vitro* the virus-like particles Aβ(2-6)E2₆₀ₘₑᵣ e Aβ(1-11)E2₆₀ₘₑᵣ.

Therefore, the object of the invention is a multimeric protein molecule wherein each monomer comprises the sequence SEQ ID NO. 6 or an amino acidic sequence included in the sequence SEQ ID NO. 6 and an amino acidic sequence that constitutes the catalytic domain of the E2 monomer of the pyruvate dehydrogenase.

Preferably, the sequence comprised in the SEQ ID NO. 6 is the SEQ ID. NO. 5.

Preferably the monomer E2 of the pyruvate dehydrogenase is that of *Geobacillus stearothermophilus.*

More preferably the catalytic domain of the E2 monomer comprises the amino acidic sequence of SEQ ID NO. 7.

In one embodiment the multimeric protein molecule of the invention is a 60mer.

Preferably, the molecule is able to induce an antibody response against the beta amyloid peptide.

Yet more preferably, the multimeric protein molecule of the invention is for medical use.

In particular, for use in the treatment and/or prevention of neurodegenerative diseases.

Preferably for use in the treatment and/or prevention of Alzheimer's disease and/or of amyloid deposition diseases.

An object of the invention is the use of the multimeric protein molecule of the invention for the preparation of a medicament for the treatment and/or prevention of neurodegenerative diseases.

In particular, for the treatment and/or prevention of Alzheimer's disease and/or of amyloid deposition diseases.

An object of the invention is a pharmaceutical and/or immunizing composition comprising the molecule of the invention and pharmacologically acceptable excipients and/or diluents.

Further described herein is a method for immunizing a patient comprising the administration of the molecule of the invention in which an antibody response to the beta amyloid protein is obtained.

Preferably, the antibody response has a prevalence of IgG1. More preferably, the antibody response to the beta amyloid protein is of the TH2 type.

In the present invention, the beta amyloid peptide comprises the aggregate as well as the pre-aggregate and non aggregate form.

An embodiment of the invention is now described, premising that said description is not restrictive with respect to variations a person having ordinary skill in the related art may implement. We shall then present some examples that show the essential characteristics of the operation of the invention, specifying that said examples are not to be construed in a restrictive sense. The examples are illustrated by the following non limiting figures.

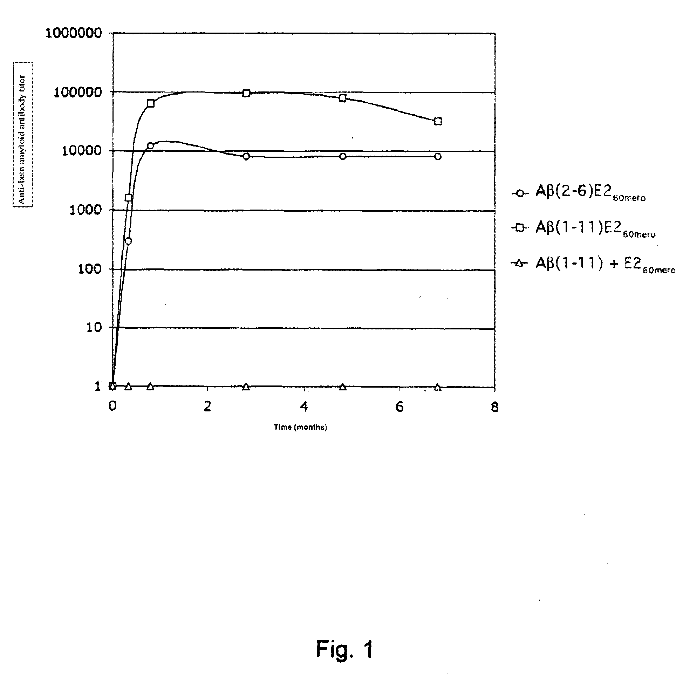

**Fig. 1****:** The chart represents the variation over time of the average antibody titer observed in 3 groups of BALB/c mice, which on day 0 and on day 14 received an injection containing one of the following compositions: Aβ(2-6)E2₆₀ₘₑᵣ (group represented with the circle symbol), Aβ (1-11)E2₆₀ₘₑᵣ (group represented with the square symbol), the synthetic peptide Aβ (1-11) mixed with wild type E2₆₀ₘₑᵣ (group represented with the triangle symbol).

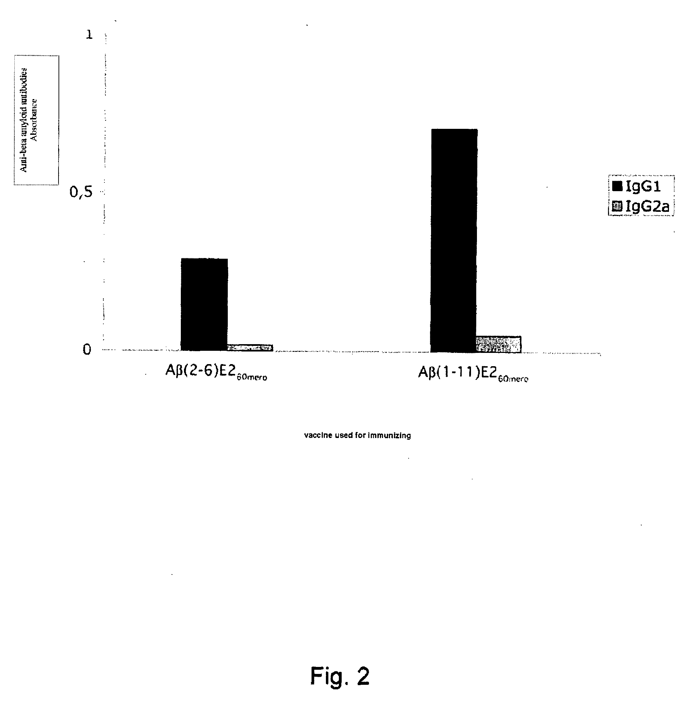

**Fig. 2****:** The chart shows the absorbance obtained in an ELISA test that measured the anti IgG1 isotype or IgG2a antibodies that recognize the pre-aggregate beta amyloid peptide 1-42, in the serum of BALB/c mice subjected to an immunization and booster treatment with VLP Aβ(1-1)E2₆₀ₘₑᵣ or Aβ(2-6)E2₆₀ₘₑᵣ. In the illustrated experiment, the serums of the two groups of immunized mice, each comprising 5 animals, obtained 10 days after the second antigen injection, were mixed to form pools.

### METHODS

### CONSTRUCTION OF THE pET(2-6)E2 E pET(1-11)E2 VECTORS

To obtain the expression of beta amyloid peptides as N-terminal fusions at the E2DISP core, the authors built, starting from the pETE2DISP vector, (Domingo GJ, Orru' S, Perham RN, J Mol Biol. 2001 Jan 12;305(2):259-67), the pET(2-6)E2 and pET(1-11)E2 VECTORS.

For this purpose, we used the following synthetic oligonucleotides:
SEQ ID NO. 1 :5'-CATGGCTGAATTCCGTCACC-3'
SEQ ID NO. 2: 5'-CCGGGGTGACGGAATTCAGC-3'
SEQ ID NO. 3: 5'-CATGGACGCTGAATTCCGTCACGACTCCGGTTACGAAC-3'
SEQ ID NO. 4: 5'-CCGGGTTCGTAACCGGAGTCGTGACGGAATTCAGCGTC-3' SEQ ID NO. 1 and SEQ ID NO. 2 are complementary and encode the peptide Aβ(2-6); SEQ ID NO. 3 and SEQ ID NO. 4 are complementary and encode the peptide Aβ(1-11). Moreover, the oligonucleotides contain the sequences recognized by the NcoI and XmaI restriction enzymes. The complementary oligonucleotides were hybridized with each other (SEQ ID NO. 1 with SEQ ID NO. 2, SEQ ID NO. 3 with SEQ ID NO. 4) and inserted in the vector pETE2DISP digested with the enzymes NcoI and XmaI, by ligase with the enzyme T4 DNA ligase. The re-circularized plasmid was selected transforming *E.coli* TG1 cells, which were selected on LB-agar plates, containing ampicillin. The correctness of the sequences of the pET(2-6)E2 and pET(1-11)E2 plasmids was confirmed by DNA sequencing.

### EXPRESSION AND PURIFICATION OF THE Aβ(2-6)E2 AND Aβ(1-11)E2 PROTEINS

To produce the VLP Aβ(2-6)E2₆₀ₘₑᵣ and Aβ(1-11)E2₆₀ₘₑᵣ, *E. coli* BL21 (DE3) cells transformed with the pET(2-6)E2 or pET(1-11)E2 plasmids were grown in a LB medium containing 100µg/ml of ampicillin at 37 °C up to an O.D. 0.6 (wavelength 600 nm), and then induced overnight at 30°C with 1 mM IPTG (Isopropyl-beta-thiogalactopyranoside, Inalco, 1758-1400). The bacteria were harvested by centrifuging at 6000 rpm for 10 minutes, resuspended in the A buffer (20 mM Tris-HCl pH8.5, 10 mM EDTA, 1 mM PMSF) containing 0.1 mg/ml lysozyme, placed in ice for 20 minutes, and lysated by sonication. The lysates were clarified by centrifuging at 10000 rpm for 1 hour, and the supernatant was subjected to fractioning with ammonium sulfate. The proteins precipitated at a saturation ranging between 35% and 65% were re-dissolved in the A buffer, dialyzed extensively against the same buffer, and applied on a Pharmacia Hiload 16/10Q-Sepharose HP anion exchange column, previously balanced with the B buffer (20 mM Tris-HCl pH 8.5, 1 mM EDTA). The Aβ(2-6)E2₆₀ₘₑᵣ and Aβ(1-11)E2₆₀ₘₑᵣ proteins were eluted with a linear gradient of 0-1 M in the B buffer, at a flow rate of 1ml/minute on 200 ml. The fractions containing the recombinant proteins were joined together, concentrated using a Centriprep30 (Amicon), and loaded on a Pharmacia Superose6 gel filtration column balanced with 50mM potassium phosphate pH 8.5. The proteins were eluted from the column at the characteristic elution volume of the 60mer. Protein concentration was determined with the Coomassie dye-binding method (Bradford assay). The purified VLP Aβ(2-6)E2₆₀ₘₑᵣ and Aβ(1-11)E2₆₀ₘₑᵣ were preserved at -80°C.

### IMMUNIZATIONS

The mice were immunized intraperitoneally with 200µl of a 1:1 mixture of antigen and adjuvant, in particular with a such quantity of VLP as to contain 5µg of the peptide of interest. In the first injection, the complete Freund's adjuvant (CFA) was used; in the subsequent injection, the incomplete Freund's adjuvant (IFA) was used. Blood was drawn from the tail 10 days after each immunization and at the indicated times.

### ELISA

The wells of a 96-well Nunc-Immuno plate were covered with streptavidin evaporation at 37°C overnight. The wells were blocked with 0.5% BSA in 20mM TrisHCl pH 7.3, 120mM NaCl, covered with 50ng of peptide biotinylate, incubated with mouse serum diluted in 0.25% BSA, 20mM TrisHCl pH 7.3, 0.5M NaCl, 0.05% Tween 20, and detected with a mouse anti-IgG antibody conjugated with peroxidase (SIGMA A-2554). All incubations were done at 37°C for 1 hour, and after each pass the wells were washed twice with EWB (20mM TrisHCl pH 7.3, 130mM NaCl, 0.05% Tween 20) and once with TBS (20mM TrisHCl pH 7.3, 0.5M NaCl). The wells were incubated for 45 minutes at ambient temperature with 0.4mg/ml O-phenylenediamine dihydrochloride dissolved in 30mM citric acid, 70mM Na₂HPO₄, 0.8mM H₂O₂. Absorbance was read at 492 nm, after blocking color development with sulfuric acid 0.8M. Each serum was tested against the synthetic peptides 1-11 and 23-29 of the beta amyloid. When indicated, the serums were also tested against the pre-aggregated synthetic beta amyloid 1-42. The beta amyloid 1-42 was made to aggregate at 37°C for 3 days, at the concentration of 1.1mg/ml in NaCl 0.1M, NaHPO₄ 0.05M pH7.5. The wells of the ELISA plate were covered with 1µg/well of sonicated amyloid aggregate, in PBS, by incubation at 4°C for 4 hours.

To determine the isotype of the antibodies against the beta amyloid, the wells of an ELISA plate were covered with pre-aggregated beta amyloid, and the following BD Biosciences Pharmingen rat monoclonal antibodies were used: anti mouse IgM 550588, anti mouse IgG₁ 559626, anti mouse IgG₂ₐ 553391, anti mouse IgG_{2b} 550333, anti mouse IgG₃ 553401. To detect the biotinylated antibodies, streptavidin conjugated with peroxidase 554066 was used. The titer of a serum was defined as the highest dilution that gives a greater value of absorbance than double the background value obtained against an irrelevant antigen.

### EXAMPLES

### EXAMPLE 1

After a single injection of the VLP Aβ(1-11)E2₆₀ₘₑᵣ of Aβ(2-6)E2₆₀ₘₑᵣ in CFA in two groups of Balb/c mice (5 mice per group) the authors of the present invention observed, after 10 days from the injection, a measurable anti-beta-amyloid response in all injected mice. The 5 control mice, injected with a mixture in CFA of the synthetic peptide Aβ(1-11) and of E2₆₀ₘₑᵣ wild type, not physically bound, did not produce antibodies against beta amyloid.

After a booster injection made 14 days after the first immunization using, for each mouse, the same antigen as the first injection, with the IFA adjuvant, the average anti-bet-amyloid titer measured after 10 days from the booster was 1:12000 in mice immunized with Aβ(2-6)E2₆₀ₘₑᵣ, and 1:64000 in mice immunized with Aβ(1-11)E2₆₀ₘₑᵣ, whilst not anti-beta-amyloid antibodies were produced in the control mice (Fig. 1).

### EXAMPLE 2

The authors of the present invention analyzed the persistence of the anti beta amyloid antibodies in the serum of mice that received two injections of Aβ(1-11)E2₆₀ₘₑᵣ or Aβ(2-6)E2₆₀ₘₑᵣ. They observed that six months after the booster injection, anti-beta amyloid antibodies are still observed in all vaccinated mice, and the average anti-beta amyloid antibody titer is 1:8000 in mice vaccinated with Aβ(2-6)E2₆₀ₘₑᵣ and 1:32000 in those vaccinated with Aβ(1-11)E2₆₀ₘₑᵣ (Fig. 1).

### EXAMPLE 3

The authors of the present invention measured the isotype of the anti-beta-amyloid antibodies obtained after an immunization and booster treatment with VLP Aβ(1-11)E2₆₀ₘₑᵣ or Aβ(2-6)E2₆₀ₘₑᵣ. Vaccination with the VLP Aβ(1-11)E2₆₀ₘₑᵣ or Aβ(2-6)E2₆₀ₘₑᵣ induces prevalently IgG1 antibodies, (indicating a TH2 response in mice), and a reduced quantity of IgG2a (indicating a TH1 response in mice) (Fig. 2).

### BIBLIOGRAPHY

Chackerian B., Rangel M., Hunter Z. and Peabody D. S. 2006. Vaccine. 24, 6321-6331.
Esposito M., et al., 2008. Mol Immunol. 45, 1056-1062.
Gilman S., et al., 2005. Neurology. 64, 1553-1562.
Kim H. D., Jin J. J., Maxwell J. A. and Fukuchi K. 2007. Immunol Lett. 112, 30-38.
Morgan C., et al., 2004. Prog Neurobiol. 74, 323-349.
Morgan D. and Gitter B. D. 2004. Neurobiol Aging. 25, 605-608.
Weksler M. E., Gouras G., Relkin N. R. and Szabo P. 2005. Immunol Rev. 205, 244-256.
Domingo G.J., Perham R.N., EP1244775; WO0142439
Domingo GJ, Orru' S, Perham RN, J Mol Biol. 2001 Jan 12;305(2):259-67

## Claims

1. A multimeric protein molecule wherein each monomer comprises an Aβ peptide and the catalytic domain of an E2 monomer of pyruvate dehydrogenase, wherein the Aβ peptide consists of SEQ ID NO:5 or SEQ ID NO:6.

2. The multimeric protein molecule according to claim 1, wherein the E2 monomer of pyruvate dehydrogenase is that of *Geobacillus stearothermophilus.*

3. The multimeric protein molecule according to claim 2, wherein the catalytic domain of the E2 monomer comprises the amino acid sequence of SEQ ID NO. 7.

4. The multimeric protein molecule according to any of claims 1 to 3, being a 60mer.

5. The multimeric protein molecule according to any of claims 1 to 4, which is able to induce an antibody response against the beta amyloid peptide.

6. The multimeric protein molecule according to claim 5, wherein the antibody response has a prevalence of IgG1.

7. The multimeric protein molecule according to claim 5, wherein the antibody response to the beta amyloid protein is of the TH2 type.

8. The multimeric protein molecule according to any of claims 1 to 7, for medical use.

9. The multimeric protein molecule for use according to claim 8, wherein the medical use is the treatment and/or prevention of neurodegenerative diseases.

10. The multimeric protein molecule for use according to claim 9, wherein the neurodegenerative disease is Alzheimer's disease and/or amyloid deposition diseases.

11. Use of the multimeric protein molecule according to any of claims 1 to 7, for the preparation of a medicament for the treatment and/or prevention of neurodegenerative diseases.

12. The use according to claim 11, wherein the medicament is for the treatment and/or prevention of Alzheimer's disease and/or of amyloid deposition diseases.

13. A pharmaceutical and/or immunizing composition comprising the multimeric protein molecule according to claim 8 and pharmacologically acceptable excipients and/or diluents.

## Patentansprüche

1. Multimeres Proteinmolekül, bei dem jedes Monomer ein Aβ-Peptid und die katalytische Domäne eines E2-Monomers von Pyruvatdehydrogenase umfasst, wobei das Aβ-Peptid aus SEQ ID NO:5 oder SEQ ID NO:6 besteht.

2. Multimeres Proteinmolekül nach Anspruch 1, bei dem das E2-Monomer von Pyruvatdehydrogenase dasjenige von *Geobacillus stearothermophilus* ist.

3. Multimeres Proteinmolekül nach Anspruch 2, bei dem die katalytische Domäne des E2-Monomers die Aminosäuresequenz von SEQ ID NO. 7 umfasst.

4. Multimeres Proteinmolekül nach einem der Ansprüche 1 bis 3, das ein 60mer ist.

5. Multimeres Proteinmolekül nach einem der Ansprüche 1 bis 4, das eine Antikörperreaktion gegen das beta-Amyloidpeptid induzieren kann.

6. Multimeres Proteinmolekül nach Anspruch 5, bei dem die Antikörperreaktion eine Prävalenz von IgG1 aufweist.

7. Multimeres Proteinmolekül nach Anspruch 5, bei dem die Antikörperreaktion auf das beta-Amyloidprotein vom TH2-Typ ist.

8. Multimeres Proteinmolekül nach einem der Ansprüche 1 bis 7 für eine medizinische Verwendung.

9. Multimeres Proteinmolekül zur Verwendung nach Anspruch 8, wobei die medizinische Verwendung die Behandlung und/oder Prävention von neurodegenerativen Erkrankungen ist.

10. Multimeres Proteinmolekül zur Verwendung nach Anspruch 9, wobei es sich bei der neurodegenerativen Erkrankung um Alzheimer-Krankheit und/oder Amyloidabscheidungserkrankungen handelt.

11. Verwendung des multimeren Proteinmoleküls nach einem der Ansprüche 1 bis 7 zur Herstellung eines Medikaments zur Behandlung und/oder Prävention von neurodenerativen Erkrankungen.

12. Verwendung nach Anspruch 11, wobei das Medikament zur Behandlung und/oder Prävention von Alzheimer-Krankheit und/oder von Amyloidabscheidungserkrankungen dient.

13. Pharmazeutische und/oder immunisierende Zusammensetzung, die das multimere Proteinmolekül nach Anspruch 8 und pharmakologisch verträgliche Träger und/oder Verdünnungsmittel umfasst.

## Revendications

1. Molécule de protéine multimère dans laquelle chaque monomère comprend un peptide Aβ et le domaine catalytique d'un monomère E2 de la pyruvate déshydrogénase, dans laquelle le peptide Aβ est constitué de SEQ ID NO : 5 ou SEQ ID NO : 6.

2. Molécule de protéine multimère selon la revendication 1, dans laquelle le monomère E2 de la pyruvate déshydrogénase est celui de *Geobacillus stearothermophilus.*

3. Molécule de protéine multimère selon la revendication 2, dans laquelle le domaine catalytique du monomère E2 comprend la séquence d'acides aminés de SEQ ID NO : 7.

4. Molécule de protéine multimère selon l'une quelconque des revendications 1 à 3, étant un 60-mer.

5. Molécule de protéine multimère selon l'une quelconque des revendications 1 à 4, qui est capable d'induire une réponse en anticorps contre le peptide bêta-amyloïde.

6. Molécule de protéine multimère selon la revendication 5, la réponse en anticorps présentant une prévalence d'IgG1.

7. Molécule de protéine multimère selon la revendication 5, la réponse en anticorps contre la protéine bêta-amyloïde étant du type TH2.

8. Molécule de protéine multimère selon l'une quelconque des revendications 1 à 7, pour une utilisation médicale.

9. Molécule de protéine multimère pour une utilisation selon la revendication 8, l'utilisation médicale étant le traitement et/ou ou la prévention de maladies neurodégénératives.

10. Molécule de protéine multimère pour une utilisation selon la revendication 9, la maladie neurodégénérative étant la maladie d'Alzheimer et/ou des maladies dues à des dépôts d'amyloïde.

11. Utilisation de la molécule de protéine multimère selon l'une quelconque des revendications 1 à 7, pour la préparation d'un médicament destiné au traitement et/ou à la prévention de maladies neurodégénératives.

12. Utilisation selon la revendication 11, dans laquelle le médicament est destiné au traitement et/ou à la prévention de la maladie d'Alzheimer et/ou de maladies dues à des dépôts d'amyloïde.

13. Composition pharmaceutique et/ou immunisante comprenant la molécule de protéine multimère selon la revendication 8 et des excipients et/ou des diluants pharmacologiquement acceptables.
